# EUROPEAN PATENT SPECIFICATION

(11) **EP 3 927 259 B1**
(45) Date of publication and mention of the grant of the patent: **05.06.2024**
(21) Application number: 20758635.5
(22) Date of filing: 18.02.2020
(51) Int. Cl.: A61B 1/04, A61B 17/34, A61B 90/10, A61B 90/11, A61B 34/20, A61B 17/00, A61B 1/06, A61B 1/313, A61B 90/30

(54) **CANNULA AND OBTURATOR WITH A TRANSPARENT TIP WITH AN OPAQUE COMPONENT**
KANÜLE UND OBTURATOR MIT EINER DURCHSICHTIGEN SPITZE MIT EINER LICHTUNDURCHLÄSSIGEN KOMPONENTE
CANULE ET OBTURATEUR AYANT UNE POINTE TRANSPARENTE AVEC UN COMPOSANT OPAQUE

(30) Priority: 22.02.2019 US 201962809445 P
(43) Date of publication of application: 29.12.2021
(73) Proprietor: Rebound Therapeutics Corporation, Irvine, CA 92618 (US)
(72) Inventor: DAVIS, Peter, G., Irvine, CA 92618 (US); TSUKASHIMA, Ross, Irvine, CA 92618 (US); FULLER, Donald, Joseph, Irvine, CA 92618 (US)
(74) Representative: Nordic Patent Service A/S
(86) International application number: PCT/US2020/018643
(87) International publication number: WO 2020/172165

(56) References cited:
- EP-A1- 2 921 121
- US-A1- 2008 086 160
- US-A1- 2010 137 895
- US-A1- 2014 249 371
- US-A1- 2014 275 771
- US-A1- 2015 272 617
- US-A1- 2017 258 494
- US-A1- 2018 206 714
- US-A1- 2018 206 714

## Description

This application claims priority to U.S. Provisional Application 62/809,445, filed February 22, 2019, which is pending.

### Field of the Inventions

The inventions described below relate to the field of cannula systems for minimally invasive surgery.

### Background of the Inventions

In our previous U.S. patent application US2018/206714A1, we disclose a cannula and camera system with an obturator comprising a small diameter shaft and larger diameter, transparent obturator tip that obturates the cannula at its distal end. Using the system, a surgeon can advance the cannula into the brain while viewing tissue distal to the obturator tip, through the obturator tip. The obturator tip may reverse the image of the tissue distal to the obturator tip, presenting at its proximal surface an image of tissue distal to the tip (at its distal surface) which is reversed, depending on its construction. US2010/137895A1 discloses an obturator and endoscope system in which the obturator is isodiametric along its entire length. The obturator is hollow and fits tightly in the cannula, and visualization is provided with an endoscope disposed within the entire length of the obturator. The distal end of the endoscope abuts an imaging transmitting member, such that there is no image reversal. EP2921121A1 discloses a similar trocar and obturator system for use in the abdomen, with the additional feature that the tip of the hollow obturator shaft includes an aperture for use in insufflating the abdomen.

The invention is defined by independent claim 1. Further embodiments are defined by the dependent claims.

The devices and methods described below provide for improved visualization of the brain during minimally invasive surgery. The device comprises a cannula system with a camera mounted on the proximal end of the cannula with a view into the cannula lumen and the tissue within and below the lumen, and a obturator comprising a narrow shaft and a larger distal tip, which is transmissive to visible light (transparent or translucent), so that the tissue distal to the tip is at least partially visible, through the tip, from the proximal end of the cannula. The obturator tip preferably has a pointed, acutely conical distal tip, and includes an optically opaque component, such as a rod, disposed along the central axis of the obturator tip, which serves to prevent image reversal.

The small cross-section obturator shaft is much smaller than the inner diameter of the cannula, affording a sizable annular or circular space between the shaft and the cannula wall to provide good visibility (from the camera) of the proximal surface of the obturator tip. Lights, if necessary, may be provided in the cannula to illuminate the distal end of the obturator tip and cannula or tissue near the distal end of the cannula (lighting may instead be provided from a source outside the assembly, or from lights mounted on the proximal end of the cannula or any combination of the foregoing). Light reflected by tissue near the distal surface of the obturator tip passes through the obturator and out of the proximal surface of the obturator tip, so that a surgeon inserting or manipulating the assembly can easily see that the obturator tip is near brain tissue (which is white to gray) or blood (which is red to black).

### Brief Description of the Drawings

Figure 1 illustrates the head of a patient with an area requiring surgical intervention.
Figures 2 through 5 illustrate the cannula and obturator, in which the obturator is provided with an opaque component within a transparent tip.

### Detailed Description of the Inventions

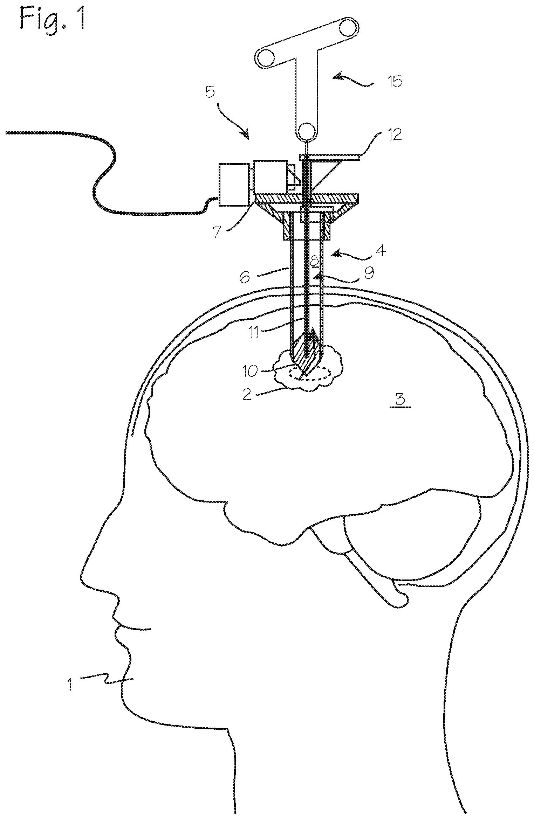

Figure 1 illustrates a patient 1 with a blood mass 2 in the brain 3 that necessitates surgical intervention. A cannula 4 has been inserted into the brain, with the distal end of the cannula proximate the blood mass. (Though illustrated in the context of brain surgery, the system can be used in any surgery.) A camera 5 is mounted on the proximal rim of the cannula, with a portion of the camera overhanging the rim of the cannula and disposed over the lumen of the cannula, and is operable to obtain video or still images of the blood mass or other tissue at the distal end of the cannula. The cannula comprises a cannula tube 6, with a distal end 6d adapted for insertion into the body of the patient, and the proximal end 6p which remains outside the body during use. The camera 5 is mounted on the proximal end 6p of the cannula tube via a mounting structure 7 secured to the proximal end of the cannula. The camera, which may include or be fitted with a prism, a reflector or other mirror structure or optical element, overhangs the lumen 8 of the cannula tube. If the camera is small compared to the cannula lumen, the camera may be used without the prism or reflector, and may be oriented with its viewing axis aligned along the long axis of the cannula.

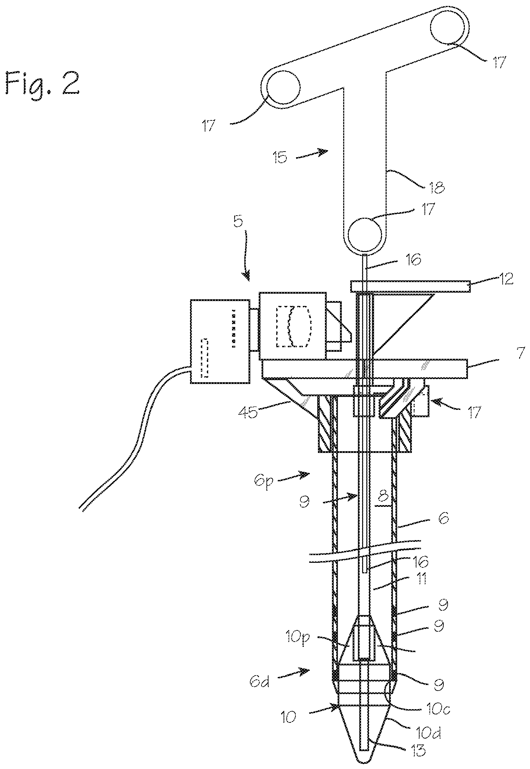

Figures 1 and 2 also illustrate the obturator 9. The obturator comprises the obturator tip 10, shaft 11 and a handle 12. The obturator tip is preferably a solid structure with a conically convex distal surface 10d, a conically convex proximal surface 10p, and an axially short circumferential surface 10c. The tip, in the region of the circumferential surface, has an outer diameter (a transverse diameter, along a plane perpendicular to the long axis of the cannula, and corresponding to the transverse cross-sectional diameter of the cannula) that closely matches the inner diameter of the cannula, but allows easy longitudinal translation of the tip through the lumen of the cannula. The tip, configured as shown in Figure 1, will act as a lens, such that light rays (represented the arrows) are refracted through the tip, and bent such that any "image" passing through the tip, when formed as illustrated, may be reversed. The tip may have a rectilinear longitudinal cross-section, with a central cylindrical portion and distal and proximal conical portions, as illustrated, or a more rounded cross-section. The distal taper preferably ends in an acutely pointed tip which is preferred for use in the brain. As illustrated, the preferred conical tip has an apex angle of about 35° to 45°. The sharpened distal tip extending distally of the cannula tube facilitates advancement of the assembly through brain tissue. The distal surface and proximal surface need not be symmetric about the longitudinal axis, or symmetric about a transverse axis. For example, the distal surface may be pointed, with a rectilinear cross-section, while the proximal surface is pointed, rounded, or flat. Though not preferred for use in the brain, the distal surface of the obturator tip may be blunt, such as spherical or spheroid, and the entire obturator tip may be formed as a sphere, a spheroid, a prolate spheroid (a football, rugby ball), and oblate spheroid, or an ovoid (egg-shaped).

The obturator tip is optically transmissive, not optically opaque, and may be optically transparent or optically translucent. The transmittance of the tip need only be adequate, in the visible spectrum, to pass the color of tissue in contact with the distal surface, given the brightness of any illumination provided by the light sources, to provide enough transmitted light to the camera and/or eye of the surgeon to allow the color of tissue around the distal tip to be discerned from light transmitted through the proximal surface of the tip. The obturator tip has a transverse cross-section closely matching a transverse cross-section of the cannula, and is slidable within the cannula tube, and positionable within the cannula tube such that the proximal end of the obturator shaft extends proximally out of the cannula proximal end while the tapered distal tip extends out of the cannula distal end. The obturator shaft has a transverse cross-section smaller than the lumen of the cannula and smaller that the transverse cross-section of the obturator tip, so that the proximal surface of the obturator tip is visible from the proximal end of the cannula, through the lumen, when the obturator tip is disposed within the cannula such that the tapered distal tip extends out of the cannula distal end.

The tip may be made of glass, silica, acrylic, polycarbonate, silicone, nylon, polyamides or copolymers or any other material suitable for use in a medical device. The obturator tip surface may be polished or frosted. The obturator tip may optionally comprise radiopaque substances (elements or compounds such as platinum particles, for example) to render the tip radiopaque, so that it appears distinctly under fluoroscopy during surgery. The obturator tip may optionally comprise sensors such as pH sensors, impedance sensors, force sensors, glucose sensors, etc., to assist in detecting a blood mass or CSF and distinguishing them from surrounding brain tissue.

The proximal surface of the tip, which tapers to a small diameter in the proximal direction, also provides for clearance of the tip when the obturator must be removed to make room for other devices, despite the position of the camera assembly at the distal end of the cannula tube.

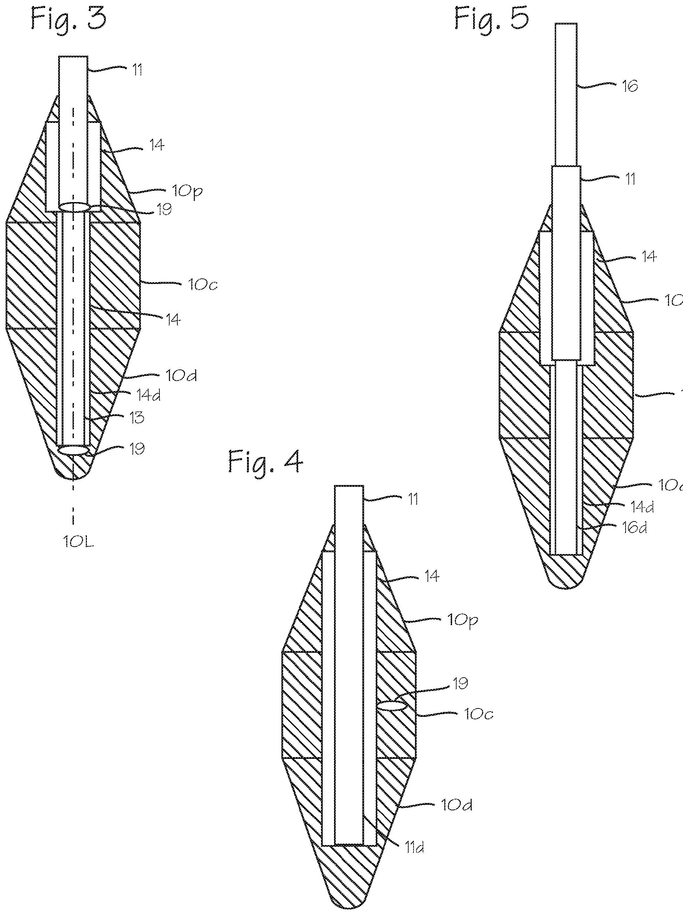

As shown in Figures 2 through 5, the obturator tip is modified by the inclusion of an opaque component or components, such as a rod 13 or any opaque substance which serves to prevent image reversal, disposed along the central axis of the obturator tip. The opaque component extends through the central cylindrical portion defined by the circumferential surface 10c and much of the distal tip of the obturator tip (that is, the distal conical portion or the portion bounded by the conically convex distal surface 10d). The opaque component is disposed along the central long axis 10L of the obturator tip, and is preferably coaxial with the obturator shaft, leaving the periphery of the obturator tip clear for maximum passage of light, and is preferably a single discrete component or single compact mass of substance. Due to the presence of this opaque component, the lensing effect of the obturator tip will be disrupted, and images of tissue on the left side of the distal tip will appear to the surgeon on the left side of the proximal surface, and images of tissue on the right side of the distal tip will appear to the surgeon on the right side of the proximal surface, etc. Also shown in Figures 2 and 3, the proximal conical section of the obturator tip includes a bore 14 which accommodates the distal end of the obturator shaft. This bore can terminate distally within the proximal conical section, or within the central cylindrical section, or even within the distal conical section. The opaque component 13 is disposed within a bore as well, and this additional bore 14d may be of smaller diameter than the bore that accommodates the shaft 11, or it may be the same diameter (and may be formed as a continuation of the shaft bore 14 or may be formed from the distal surface such that it is blind to the shaft bore). The opaque component may be provided as a component 13 separate from the obturator shaft 11, as shown in Figure 3, with a transverse dimension (a radial diameter, for example, if the radial cross-section is circular) which is smaller than the transverse dimension of the obturator shaft 11. In embodiments in which the bore which accommodates the obturator shaft terminates in the distal conical section, the obturator shaft distal end 16d may terminate also in the distal conical section, and the distal tip of the obturator shaft may serve as the optically opaque component 13, such that the opaque component is not a component separate from the obturator shaft 11. This is illustrated in Figure 4, in which the obturator tip comprises a proximal portion 10p and a central cylindrical portion 10c, and the tapered distal tip 10d is a distal conical portion, and the obturator further comprises a bore 14, 14d extending from a proximal-most extent of the proximal portion, distally into the central cylindrical portion, and in which the distal end 10d of the shaft is disposed within the bore, where the bore has a length such that, when proximal end of the obturator shaft extends proximally out of the cannula proximal end the tapered distal tip extends out of the cannula distal end, the bore 14, 14d terminates distally at a point distal to the distal end of the cannula tube. In this embodiment, the opaque component may comprise the distal end 11d of the shaft. The opaque component/distal end of the obturator shaft may have a transverse dimension which is smaller than the transverse dimension of the obturator shaft 11, or, as shown, it may have the same transverse dimension as the obturator shaft.

In embodiments in which the shaft bore terminates in the proximal conical section, or central cylindrical section, the neuro-navigation stylet distal tip 16d may extend distally beyond the shaft bore 14 (the larger bore which accommodated the shaft 11), and extend into the distal conical section (in the smaller bore 14d extending beyond the shaft bore, of smaller diameter that the shaft bore), for example into a smaller diameter bore the extends distally from the shaft bore, to serve as the opaque component. This is shown in Figure 5, in which the distal end of the navigation stylet extends well past the open distal end of the cannula tube, and into the distal conical section at the tip of the obturator which, when assembled with the cannula tube, extends past the open distal end of the cannula tube. In this embodiment, the neuro-navigation stylet has a distal end a proximal end, and is sized and dimensioned for insertion in a lumen of the obturator shaft, such that when proximal end of the obturator shaft extends proximally out of the cannula proximal end the tapered distal tip extends out of the cannula distal end, and the neuro-navigation stylet is disposed within the lumen of the obturator shaft, the distal end of the neuro-navigation stylet terminates distally at a point distal to the distal end of the cannula tube. The distal end of the neuro-navigation stylet may serve as the opaque component comprises.

The shaft 11 may be a solid rod or a tube, with a small diameter, or transverse cross-section, compared to the cannula lumen and the obturator tip, so that the tip proximal surface can be viewed from the cannula proximal end. If provided as a tube, the lumen of the shaft may accommodate a neuro-navigation stylet or probe 15 with markers detectable by the neuro-navigation system, useful for guidance of the assembly into the brain. The rod 16 of the neuro-navigation stylet may be inserted into the lumen of tubular shaft, as shown, so that the assembled cannula, obturator and stylet may be tracked by a neuro-navigation system, through tracking of the markers 17 on a frame 18 to aid in accurate placement of the distal tip of the assembly. The shaft 11 may also accommodate a neuro starburst connection. In embodiments which include a neuro-navigation stylet, the distal tip of the stylet can terminate at any point within the obturator shaft, and may terminate proximal to the obturator tip, or within the obturator tip at a point proximal to the distal edge of the cannula or distal to the distal edge of the cannula.

In use, a surgeon will assemble the cannula tube, obturator, and, optionally, the neuro-navigation stylet for insertion into the body of a patient to gain access to a surgical workspace. For example, the method may entail providing a cannula system comprising the cannula tube, the obturator and the neuro-navigation stylet and assembling the cannula tube, obturator and neuro-navigation stylet such that the obturator is disposed within the cannula tube with the obturator tip extending distally from the cannula distal end and the distal end of end the neuro-navigation stylet extends distally from the obturator shaft and into the distal tip of the obturator tip to provide the opaque component within the distal tip, and advancing the assembled cannula system into the body while observing body tissue distal to the cannula tube, through the obturator tip, from the proximal end of the cannula tube. Where a neuro-navigation stylet is not to be used as the opaque component, the method may entail providing a cannula system comprising the cannula tube and the obturator, where the obturator tip further includes an opaque component disposed in the distal tip, and assembling the cannula tube and obturator such that the obturator is disposed within the cannula tube with the obturator tip extending distally from the cannula distal end such that the opaque component within the distal tip is disposed distally of the distal end of the cannula tube, and advancing the assembled cannula system into the body while observing body tissue distal to the cannula tube, through the obturator tip, from the proximal end of the cannula tube.

To aid in visualization, lights may be incorporated into the obturator tip to cast light on tissue distal to the tip and make it easier to see the tissue through the obturator tip from the proximal end of the cannula tube. As shown in Figure 3, a light such as an LED 19, may be disposed within the distal conical portion, and may be embedded in the material of the obturator or fixed at the bottom of the bore 14d which accommodates the opaque component. An LED may instead be disposed within the proximal portion (Figure 3) or the cylindrical portion (Figure 4), and may be embedded in the material of the obturator or fixed at the bottom of the bore 14 which accommodates the obturator shaft 11. The light source may be employed in embodiments which omit the opaque component. Wiring necessary to power the light can be disposed within or about the obturator shaft.

While the preferred embodiments of the devices have been described in reference to the environment in which they were developed, they are merely illustrative of the principles of the inventions. The elements of the various embodiments may be incorporated into each of the other species to obtain the benefits of those elements in combination with such other species, and the various beneficial features may be employed in embodiments alone or in combination with each other.

## Claims

1. A cannula system for accessing a blood mass (2) in the brain of a patient (1), said cannula system comprising:
a cannula (4) comprising a cannula tube (6) with a proximal end (6p) and a distal end (6d) and a lumen (8) extending from the proximal end (6p) to the distal end (6d); and
an obturator (9) comprising an obturator shaft (11) having a proximal end (11p) and a distal end (11d), and an obturator tip (10) disposed on the distal end (11d) of said obturator shaft (11), said obturator tip (10) being optically transmissive, said obturator tip (10) having a transverse cross-section closely matching an inner diameter of the cannula (4), said obturator tip (10) having a tapered distal tip (10d), said obturator (9) being slidable within the cannula tube (6), and positionable within the cannula tube (6) such that the proximal end (11p) of the obturator shaft (11) extends proximally out of the cannula proximal end (6p) while the tapered distal tip (10d) extends out of the cannula distal end (6d), wherein the obturator shaft (11) has a transverse cross-section smaller than the lumen (8) of the cannula (4) and smaller than the transverse cross-section of the obturator tip (10), whereby the proximal surface (10p) of the obturator tip (10) is visible from the proximal end (6p) of the cannula, through the lumen (8) and an annular space between the obturator shaft (11) and the cannula wall, when the obturator tip (10) is disposed within the cannula (4) such that the tapered distal tip (10d) extends out of the cannula distal end (6d); and
an optically opaque component (13) **characterised in that**
the optically opaque component (13) is disposed along a central longitudinal axis (10L) of the obturator tip (10), within the tapered distal tip (10d).

2. The cannula system of claim 1, wherein:
the obturator tip (10) comprises a proximal portion (10p), a central cylindrical portion, and wherein the tapered distal tip (10d) is a distal conical portion, and the obturator tip (10) further comprises a bore (14) extending from a proximal-most extent of the proximal portion (10p), distally into the central cylindrical portion, and the distal end (11d) of the shaft (11) is disposed within the bore (14), where the bore (14) has a length such that, when the proximal end (11p) of the obturator shaft (11) extends proximally out of the cannula proximal end (6p) and the tapered distal tip (10d) extends out of the cannula distal end (6d), the bore (14) terminates distally at a point distal to the distal end (6d) of the cannula tube (6).

3. The cannula system of claim 2, wherein:
the shaft (11) extends distally into the distal conical portion (10d) of the obturator tip (10), and the opaque component (13) comprises the distal end (11d) of the shaft (11).

4. The cannula system of claim 2, wherein:
the shaft (11) terminates distally in the proximal portion (10p) of the obturator tip (10), and the opaque component (13) is a component separate from the shaft (11) .

5. The cannula system of claim 1, 2, 3 or 4, further comprising:
a neuro-navigation stylet having a distal end (16d) and a proximal end, said neuro-navigation stylet sized and dimensioned for insertion in the lumen (8) of the obturator shaft (11); wherein
when proximal end (11p) of the obturator shaft (11) extends proximally out of the cannula proximal end (6p) and the tapered distal tip (10d) extends out of the cannula distal end (6d), and the neuro-navigation stylet is disposed within the lumen (8) of the obturator shaft (11), the distal end (16d) of the neuro-navigation stylet terminates distally at a point distal to the distal end (6d) of the cannula tube (6).

6. The cannula system of claim 5, wherein:
the neuro-navigation stylet terminates distally within the distal conical portion (10d) of the obturator tip (10), and the opaque component (13) comprises the distal end (16d) of the neuro-navigation stylet.

7. The cannula system of claim 1, 2, 3 or 4, wherein:
the distal conical portion (10d) has an apex angle in the range of 35 to 45 degrees.

8. The cannula system of claim 1, 2, 3 or 4, wherein:
the distal conical portion (10d) comprises a sharpened distal tip extending distally of the cannula tube (6) to facilitate advancement of the assembly through brain tissue.

## Patentansprüche

1. Kanülensystem für einen Zugang zu einer Blutmasse (2) im Gehirn eines Patienten (1), wobei das genannte Kanülensystem umfasst:
eine Kanüle (4), umfassend ein Kanülenrohr (6) mit einem proximalen Ende (6p) und einem distalen Ende (6d) und einem Lumen (8), das sich von dem proximalen Ende (6p) zu dem distalen Ende (6d) erstreckt; und
einen Obturator (9), umfassend einen Obturatorschaft (11) mit einem proximalen Ende (11p) und einem distalen Ende (11d), und eine Obturatorspitze (10), die an dem distalen Ende (11d) des genannten Obturatorschafts (11) angeordnet ist, wobei die genannte Obturatorspitze (10) optisch durchlässig ist, wobei die genannte Obturatorspitze (10) einen transversalen Querschnitt aufweist, der eng mit einem Innendurchmesser der Kanüle (4) zusammenpasst, wobei die genannte Obturatorspitze (10) eine verjüngte distale Spitze (10d) aufweist, wobei der genannte Obturator (9) innerhalb des Kanülenrohrs (6) gleiten kann und innerhalb des Kanülenrohrs (6) derart positioniert werden kann, dass sich das proximale Ende (11p) des Obturatorschafts (11) proximal aus dem proximalen Ende (6p) der Kanüle erstreckt, während sich die verjüngte distale Spitze (10d) aus dem distalen Ende (6d) der Kanüle erstreckt, wobei der Obturatorschaft (11) einen transversalen Querschnitt aufweist, der kleiner ist als das Lumen (8) der Kanüle (4) und kleiner ist als der transversale Querschnitt der Obturatorspitze (10), wodurch die proximale Fläche (10p) der Obturatorspitze (10) von dem proximalen Ende (6p) der Kanüle durch das Lumen (8) und einen ringförmigen Raum zwischen dem Obturatorschaft (11) und der Kanülenwand sichtbar ist, wenn die Obturatorspitze (10) innerhalb der Kanüle (4) derart angeordnet ist, dass sich die verjüngte Spitze (10d) aus dem distalen Ende (6d) der Kanüle erstreckt; und
eine optisch opake Komponente (13);
**dadurch gekennzeichnet, dass** die optisch opake Komponente (13) entlang einer zentralen Längsachse (10L) der Obturatorspitze (10) innerhalb der verjüngten distalen Spitze (10d) angeordnet ist.

2. Kanülensystem nach Anspruch 1, wobei:
die Obturatorspitze (10) einen proximalen Abschnitt (10p), einen zentralen zylindrischen Abschnitt umfasst, und wobei die verjüngte distale Spitze (10d) ein distaler konischer Abschnitt ist, und die Obturatorspitze (10) ferner eine Bohrung (14) umfasst, die sich aus einer am meisten proximalen Ausdehnung des proximalen Abschnitts (10p) erstreckt, distal in den zentralen zylindrischen Abschnitt, und das distale Ende (11d) des Schafts (11) innerhalb der Bohrung (14) angeordnet ist, wobei die Bohrung (14) eine solche Länge aufweist, dass, wenn sich das proximale Ende (11p) des Obturatorschafts (11) proximal aus dem proximalen Ende (6p) der Kanüle erstreckt, und sich die verjüngte distale Spitze (10d) aus dem distalen Ende (6d) der Kanüle erstreckt, die Bohrung (14) distal an einem Punkt distal von dem distalen Ende (6d) des Kanülenrohrs (6) endet.

3. Kanülensystem nach Anspruch 2, wobei:
sich der Schaft (11) distal in den distalen konischen Abschnitt (10d) der Obturatorspitze (10) erstreckt, und die opake Komponente (13) das distale Ende (11d) des Schafts (11) umfasst.

4. Kanülensystem nach Anspruch 2, wobei:
der Schaft (11) distal in dem proximalen Abschnitt (10p) der Obturatorspitze (10) endet, und die opake Komponente (13) eine Komponente getrennt von dem Schaft (11) ist.

5. Kanülensystem nach Anspruch 1, 2, 3 oder 4, ferner umfassend:
eine Neuronavigationssonde mit einem distalen Ende (16d) und einem proximalen Ende, wobei die genannte Neuronavigationssonde für ein Einsetzen in das Lumen (8) des Obturatorschafts (11) bemessen und gestaltet ist; wobei,
wenn sich das proximale Ende (11p) des Obturatorschafts (11) proximal aus dem proximalen Ende (6p) der Kanüle streckt, und sich die verjüngte distale Spitze (10d) aus dem distalen Ende (6d) der Kanüle erstreckt, und die Neuronavigationssonde innerhalb des Lumens (8) des Obturatorschafts (11) angeordnet ist, das distale Ende (16d) der Neuronavigationssonde distal an einem Punkt distal von dem distalen Ende (6d) des Kanülenrohrs (6) endet.

6. Kanülensystem nach Anspruch 5, wobei:
die Neuronavigationssonde distal innerhalb des distalen konischen Abschnitts (10d) der Obturatorspitze (10) endet, und die opake Komponente (13) das distale Ende (16d) der Neuronavigationssonde umfasst.

7. Kanülensystem nach Anspruch 1, 2, 3 oder 4, wobei:
der distale konische Abschnitt (10d) einen Scheitelwinkel im Bereich von 35 bis 45 Grad aufweist.

8. Kanülensystem nach Anspruch 1, 2, 3 oder 4, wobei:
der distale konische Abschnitt (10d) eine geschärfte distale Spitze umfasst, die sich distal aus dem Kanülenrohr (6) erstreckt, um das Vorschieben der Anordnung durch Gehirngewebe zu erleichtern.

## Revendications

1. Système de canule permettant d'accéder à une masse sanguine (2) dans le cerveau d'un patient (1), ledit système de canule comprenant :
une canule (4) comprenant un tube de canule (6) doté d'une extrémité proximale (6p) et d'une extrémité distale (6d) et un lumen (8) s'étendant de l'extrémité proximale (6p) à l'extrémité distale (6d) ; et
un obturateur (9) comprenant une tige d'obturateur (11) dotée d'une extrémité proximale (11p) et d'une extrémité distale (11d), et une pointe d'obturateur (10) disposée sur l'extrémité distale (11d) de ladite tige d'obturateur (11), ladite pointe d'obturateur (10) étant optiquement transmissive, ladite pointe d'obturateur (10) ayant une section transversale correspondant étroitement à un diamètre intérieur de la canule (4), ladite pointe d'obturateur (10) ayant une pointe distale effilée (10d), ledit obturateur (9) pouvant glisser dans le tube de canule (6), et étant positionnable dans le tube de canule (6) de manière à ce que l'extrémité proximale (11p) de la tige d'obturateur (11) s'étende proximalement hors de la canule (6p) tandis que la pointe distale effilée (10d) s'étend hors de l'extrémité distale de la canule (6d), la tige d'obturateur (11) ayant une section transversale inférieure au lumen (8) de la canule (4) et inférieure à la section transversale de la pointe d'obturateur (10), la surface proximale (10p) de la pointe d'obturateur (10) étant visible depuis l'extrémité proximale (6p) de la canule à travers le lumen (8) et un espace annulaire entre la tige d'obturateur (11) et la paroi de la canule lorsque la pointe d'obturateur (10) est disposée dans la canule (4), de sorte que la pointe distale effilée (10d) s'étend hors de l'extrémité distale de la canule (6d) ; et
un composant optiquement opaque (13), **caractérisé en ce que** le composant optiquement opaque (13) est disposé le long d'un axe longitudinal central (10L) de l'bas la pointe d'obturateur (10) dans la pointe distale effilée (10d).

2. Système de canule selon la revendication 1, dans lequel :
la pointe d'obturateur (10) comprend une section proximale (10p), une section cylindrique centrale, et la pointe distale effilée (10d) est une section conique distale, et la pointe d'obturateur (10) comprend en outre un trou (14) s'étendant depuis une extension la plus proximale de la section proximale (10p), distalement jusque dans la section cylindrique centrale, et l'extrémité distale (11d) de la tige (11) est disposée dans le trou (14), le trou (14) ayant une longueur telle que, lorsque l'extrémité proximale (11p) de la tige d'obturateur (11) s'étend proximalement hors de l'extrémité proximale de la canule (6p) et que la pointe distale effilée (10d) s'étend hors de l'extrémité distale de la canule (6d), le trou (14) finit distalement à un point distal à l'extrémité distale (6d) du tube de canule (6).

3. Système de canule selon la revendication 2, dans lequel :
la tige (11) s'étend distalement dans la section conique distale (10d) de la pointe d'obturateur (10), et le composant opaque (13) comprend l'extrémité distale (11d) de la tige (11).

4. Système de canule selon la revendication 2, dans lequel :
la tige (11) se termine distalement dans la section proximale (10p) de la pointe d'obturateur (10), et le composant opaque (13) est un composant séparé de la tige (11).

5. Système de canule selon la revendication 1, 2, 3 ou 4, comprenant en outre :
un stylet de neuro-navigation doté d'une extrémité distale (16d) et d'une extrémité proximale, ledit stylet de neuro-navigation étant taillé et dimensionné pour être inséré dans le lumen (8) de la tige d'obturateur (11) ; sachant que,
lorsque l'extrémité proximale (11p) de la tige d'obturateur (11) s'étend proximalement hors de l'extrémité proximale de la canule (6p) et que la pointe distale effilée (10d) s'étend hors de l'extrémité distale de la canule (6d), et que le stylet de neuro-navigation est disposé dans le lumen (8) de la tige d'obturateur (11), l'extrémité distale (16d) du stylet de neuro-navigation finit distalement à un point distal à l'extrémité distale (6d) du tube de canule (6).

6. Système de canule selon la revendication 5, dans lequel :
le stylet de neuro-navigation se termine distalement dans la section conique distale (10d) de la pointe d'obturateur (10), et le composant opaque (13) comprend l'extrémité distale (16d) du stylet de neuro-navigation.

7. Système de canule selon la revendication 1, 2, 3 ou 4, dans lequel :
la section conique distale (10d) a un angle apical de l'ordre de 35 à 45 degrés.

8. Système de canule selon la revendication 1, 2, 3 ou 4, dans lequel :
la section conique distale (10d) comprend une pointe distale aiguisée s'étendant distalement par rapport au tube de canule (6) pour faciliter l'avancement de l'ensemble à travers le tissu cérébral.
